# EUROPÄISCHE PATENTSCHRIFT

(11) **EP 1 047 669 B1**
(45) Veröffentlichungstag und Bekanntmachung des Hinweises auf die Patenterteilung: **06.11.2002**
(21) Anmeldenummer: 99903620.5
(22) Anmeldetag: 11.01.1999
(51) Int. Cl.: C07C 309/65, C07F 9/50

(54) **PERFLUOR-N-ALKANSULFONSÄURE-DERIVATE**
PERFLUORO-N-ALKYLSULFONIC ACID DERIVATIVES
DERIVES D'ACIDE PERFLUORO-N-ALCANESULFONIQUE

(30) Priorität: 15.01.1998 DE 19801248
(43) Veröffentlichungstag der Anmeldung: 02.11.2000
(73) Patentinhaber: MERCK PATENT GmbH, 64293 Darmstadt (DE)
(72) Erfinder: WÄCHTLER, Andreas, D-72076 Tübingen (DE); DERWENSKUS, Karl-Heinz, D-64287 Darmstadt (DE); MEUDT, Andreas, D-60529 Frankfurt (DE)
(86) Internationale Anmeldenummer: EP9900119
(87) Internationale Veröffentlichungsnummer: WO99036397

(56) Entgegenhaltungen:
- US-A- 5 399 771

## Beschreibung

Die Erfindung betrifft neue Bis(perfluor-n-alkansulfonate) der Formel I: 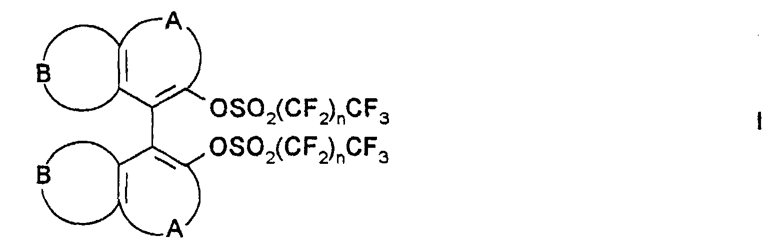 worin
- n: 3, 4, 5, 6, 7, 8 oder 9,
- A: 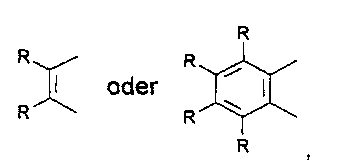
- B: -(CHR)₄-, 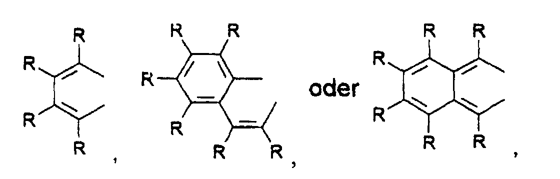 wobei nicht benachbarte Gruppen =CR- durch =N- und ferner -CHRdurch -NR-, -O- oder -S- ersetzt sein können
und
- R: Alkyl oder Alkoxy mit 1 bis 12 C-Atomen, Halogen, -CN, -CF₃, -OCF₃ oder unsubstituiertes oder einfach oder mehrfach durch Alkyl oder Alkoxy mit 1 bis 12 C-Atomen, Halogen oder -CN substituiertes Phenyl, wobei R bei mehrfachem Auftreten dieselbe oder verschiedene Bedeutungen aufweisen kann,
bedeuten.

Die Erfindung betrifft weiterhin ein Verfahren zur Herstellung der Bis(perfluor-n-alkansulfonate) der Formel I und deren Verwendung als Vorstufe für die Herstellung von chiralen Phosphan-Liganden für Übergangsmetallkatalysatoren.

Chirale Phosphan-Liganden, wie z.B. 2,2'-Bis(diphenylphosphin)-1,1'-binaphthyl (BINAP) und analoge Phosphane sind als Bestandteil von Übergangsmetallkatalysatoren, die in enantioselektiven Hydrierungen oder CC-Verknüpfungen eingesetzt werden, von großer Bedeutung. Verschiedene Wege zur Herstellung dieser Phosphane sind der Literatur bekannt. Im allgemeinen wird dabei von den entsprechenden Binaphtholen oder analogen Phenol-Derivaten ausgegangen, deren Hydroxygruppen in Abgangsgruppen überführt werden und anschließend gegen Phosphan-Reste ausgetauscht werden.

USP 5,399,771 offenbart ein Verfahren zur Herstellung von BINAP, das von enantiomerenreinem Binaphthol ausgeht, welches zunächst in das entsprechende Bis(trifluormethansulfonat) überführt wird. Anschließend wird durch Nickel-katalysierte Kopplung mit Diphenylphosphin BINAP erhalten. Nachteil dieses Verfahrens ist der hohe Preis und die im industriellen Maßstab aufwendige Handhabung des empfindlichen und äußerst aggressiven Trifluormethansulfonsäureanhydrids bei der Herstellung von Binaphtholbis(trifluormethansulfonat). Auch die Verwendung anderer Trifluormethansulfonsäurederivate wie Trifluormethansulfonsäurefluorid oder -chlorid ist durch die hohe Flüchtigkeit der Verbindungen (Kp -20°C bzw. Kp. 32°C) verfahrenstechnisch aufwendig.

Der Erfindung lag die Aufgabe zugrunde, ein industriell durchführbares Verfahren bereitzustellen, das die oben genannten Nachteile nicht aufweist und einen verfahrenstechnisch einfachen und kostengünstigen Zugang zu Verbindungen ermöglicht, die sich als Ausgangssubstanzen für die Synthese von chiralen Phosphan-Liganden für Übergangsmetallkatalysatoren eignen.

Überraschenderweise wurde gefunden, daß die Verbindungen der Formel l in einfacher Weise durch Umsetzung der jeweiligen Phenole mit den entsprechenden Perfluor-n-alkansulfonylfluoriden, -chloriden oder -anhydriden zugänglich sind und durch die Verwendung der Verbindungen der Formel l deutlich verbesserte Ausbeuten bei der Herstellung von Phosphan-Liganden für Übergangsmetallkatalysatoren erhalten werden.

Die entsprechenden längerkettigen Perfluoralkansulfonylfluoride, chloride oder anhydride sind preiswert kommerziell erhältlich oder nach bekannten Verfahren leicht herstellbar (z.B. DE 1912738, DE 4218562).

Gegenstand der Erfindung sind somit Bis(perfluor-n-alkansulfonate) der Formel l: 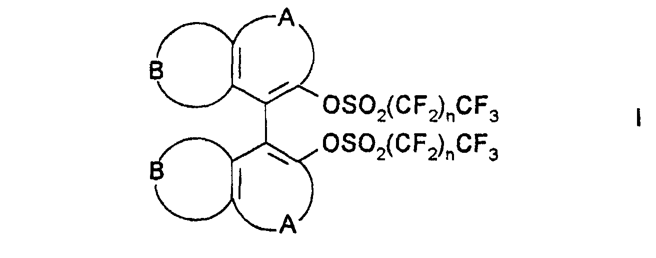 worin
A, B und n die oben angegebene Bedeutung aufweisen.

Ein weiterer Gegenstand der Erfindung ist ein Verfahren zur Herstellung der Bis(perfluor-n-alkansulfonate) der Formel 1, dadurch gekennzeichnet, daß man Verbindungen der Formel II 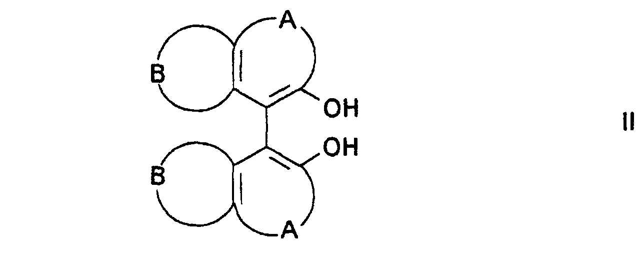 worin A und B die oben angegebene Bedeutung aufweisen, mit dem entsprechenden Perfluor-n-alkansulfonylfluorid, -chlorid oder -anhydrid in Gegenwart einer Base umsetzt.

Die nach dem erfindungsgemäßen Verfahren hergestellten Bis(perfluor-nalkansulfonate) der Formel l aus den Phenolen der Formel II, stellen insbesondere wertvolle Zwischenprodukte zur Synthese von chiralen Katalysatoren dar, wie z.B. 2,2'-Bis(diphenylphosphin)-1,1'-binaphthyl (BINAP) und analoger Verbindungen.
A bedeutet bevorzugt 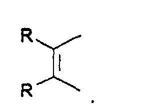 B weist vorzugsweise die Bedeutung -(CHR₂)₄- oder 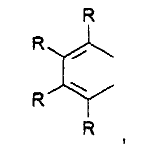 insbesondere 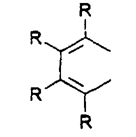 auf.
n bedeutet vorzugsweise 3, 4, 5 oder 7, insbesondere 3 oder 7. Ganz besonders bevorzugt nimmt n den Wert 3 an. R bedeutet bevorzugt Alkyl oder Alkoxy mit 1 bis 7 C-Atomen, F, Br, CN, -CF₃, -OCF₃, insbesondere - CH₃, -OCH₃, CN oder -CF₃.

Falls R in den vor- und nachstehenden Formeln einen Alkylrest oder einen Alkoxyrest bedeutet, so kann dieser geradkettig oder verzweigt sein. Vorzugsweise ist er geradkettig, hat 1, 2, 3, 4, 5, 6 oder 7 C-Atome und bedeutet demnach bevorzugt Methyl, Ethyl, Propyl, Butyl, Pentyl, Hexyl, Heptyl, Methoxy, Ethoxy, Propoxy, Butoxy, Pentyloxy, Hexyloxy oder Heptyloxy, ferner Octyl, Nonyl, Decyl, Undecyl, Dodecyl, Tridecyl, Tetradecyl, Pentadecyl, Octyloxy, Nonyloxy, Decyloxy, Undecyloxy, Dodecyloxy, Tridecyloxy oder Tetradecyloxy.

Der Rest R kann auch ein optisch aktiver organischer Rest mit einem asymmetrischen Kohlenstoffatom sein.

Vor- und nachstehend bedeutet Halogen Fluor, Chlor, Brom oder lod, vorzugsweise Fluor; Chlor oder Brom. Insbesondere bevorzugt bedeutet Halogen Fluor oder Brom.

Perfluor-n-alkansulfonate der Teilformeln 11-113 stellen besonders bevorzugte Ausführungsformen der Erfindung dar: 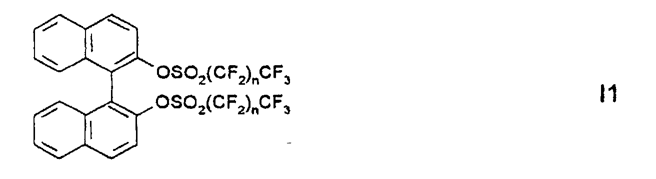 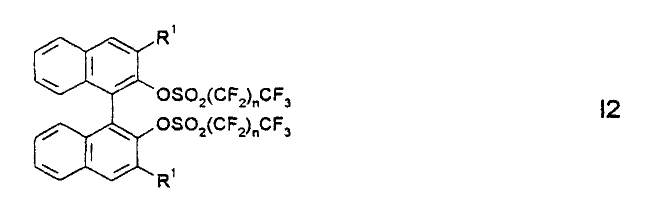 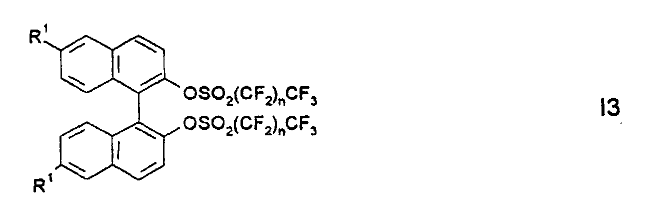 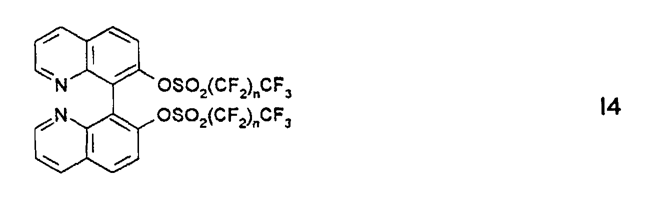 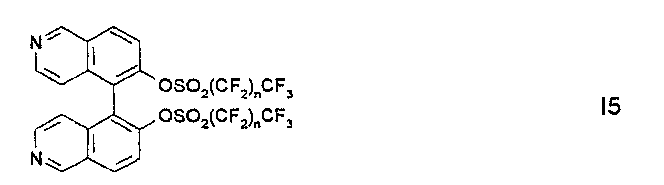 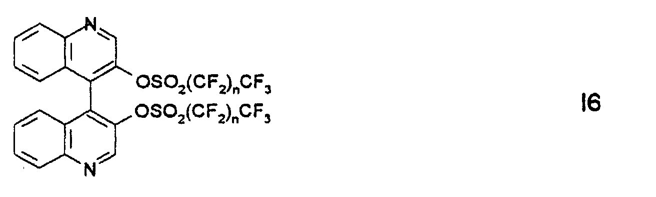 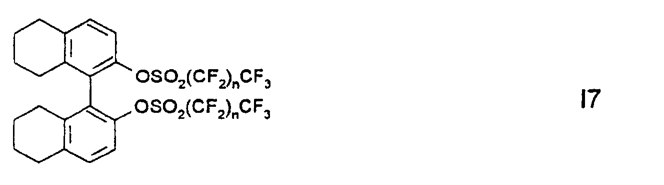 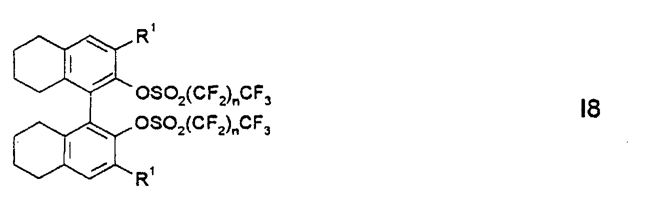 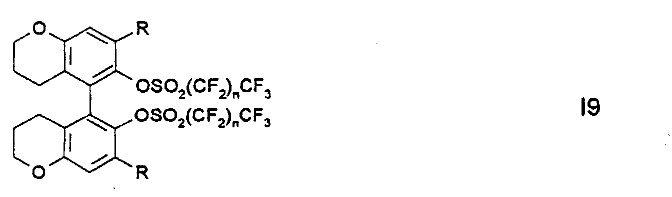 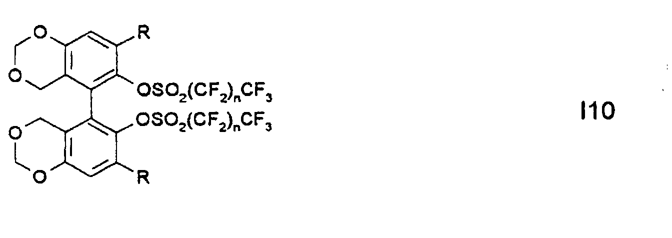 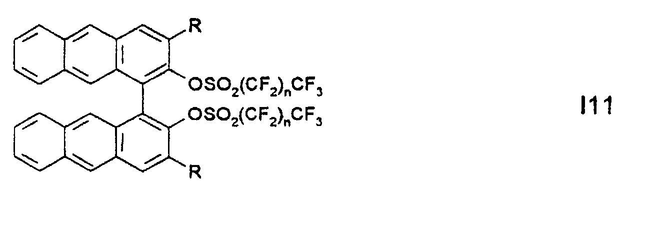 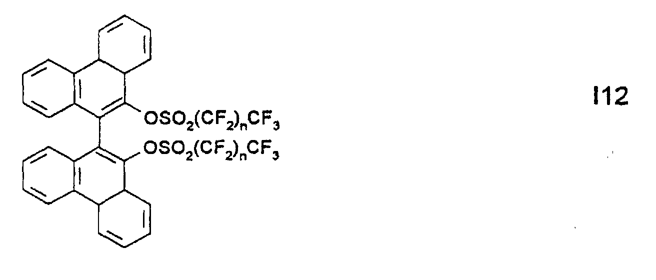 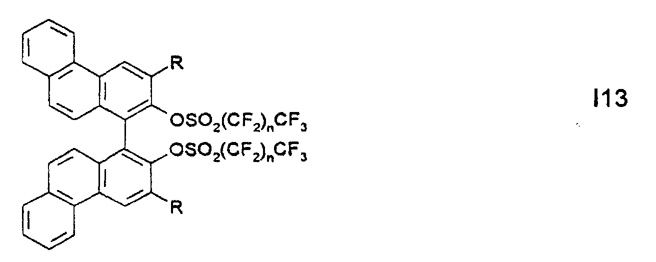 worin R und n die oben angegebene Bedeutung aufweisen und R¹ Alkyl oder Alkoxy mit 1 bis 3 C-Atomen, F, Br, CF₃ oder CN bedeutet.

Insbesondere bevorzugt sind die Verbindungen der Formeln l1, l2, l3, l7 und l8.

Die Reaktionsdurchführung des erfindungsgemäßen Verfahrens zur Herstellung der Verbindungen der Formel l ist einfach, wobei man das entsprechende Phenol-Derivat der Formel II bei Temperaturen von -30 °C bis +70°C, vorzugsweise bei -10° - +50 °C, insbesondere bei 0 bis +30 °C bei erhöhtem oder verringertem Druck, vorzugsweise bei normalem Druck mit dem entsprechenden Perfluor-n-alkansulfonylfluorid, -chlorid oder -anhydrid in Gegenwart einer Base zur Reaktion bringt. Bevorzugt wird Perfluor-n-alkansulfonylfluorid eingesetzt, um die Perfluor-n-alkansulfonate der Formel l zu erhalten. Vorzugsweise werden als Perfluor-n-alkansulfonylfluoride Nonafluor-n-butansulfonylfluorid oder Perfluor-n-oktansulfonylfluorid verwendet. Ganz besonders bevorzugt ist Nonafluorn-butansulfonylfluorid.

Das molare Verhältnis des jeweiligen Phenols der Formel II zu Perfluor-n-alkansulfonylfluorid, -chlorid oder -anhydrid beträgt für das erfindungsgemäße Verfahren im allgemeinen 1 : 2 bis 1 : 20, bevorzugt 1 : 2 bis 1 : 10. Insbesondere ist ein Verhältnis von 1 : 2 bis 1 : 5 bevorzugt.

Die Umsetzung kann in Gegenwart von gleichen molaren Mengen von Base und Perfluor-n-alkansulfonylfluorid, -chlorid oder -anhydrid oder mit einem Überschuß der jeweiligen Base ausgeführt werden.

Geeignete Basen für das erfindungsgemäße Verfahren zur Herstellung der Verbindungen der Formel l sind beispielsweise Alkali- und Erdalkalicarbonate wie z.B. Natrium-, Kalium-, Magnesium- oder Calciumcarbonat. Besonders geeignet sind Stickstoffheterocyclen, Amine oder Amidine. Vorzugsweise werden solche Stickstoffbasen eingesetzt, worin keine H-Atome direkt an ein N-Atom gebunden sind. Bevorzugte Stickstoffbasen sind Pyridine, Pyrimidine, Pyridazine, Trialkylamine oder Dialkylarylamine, wobei die Alkylreste in den Trialkylaminen und Dialkylarylaminen identisch oder unterschiedlich sein können. Insbesondere bevorzugt sind Imidazol, Pyridin, p-Dimethylaminopyridin, m-Dimethylaminopyridin, o-Dimethylaminopyridin, Pyrimidin, Trimethylamin, Triethylamin, Tripropylamin, Triisopropylamin, Dimethylanilin, Diethylanilin. Ganz besonders bevorzugt sind Pyridin, Imidazol, p-Dimethylaminopyridin, m-Dimethylaminopyridin, Trimethylamin, Triethylamin und Dimethylanilin. Es können auch Gemische der genannten Stickstoffbasen Verwendung finden.

Die Dauer der Reaktion beträgt im allgemeinen 0.1 bis 24 Stunden, bevorzugt 0.2 bis 6 Stunden.

Die Umsetzung des Phenols mit Perfluor-n-alkansulfonylfluorid, -chlorid oder -anhydrid kann in der Schmelze oder in Lösungsmitteln durchgeführt werden. Vorzugsweise wird in Gegenwart von organischen Lösungsmitteln gearbeitet.

Geeignete Lösungsmittel für das Verfahren zur Herstellung der Verbindungen der Formel l sind halogenierte Kohlenwasserstoffe wie z.B. Dichlormethan, Trichlormethan, Dichlorethylen oder Trichlorethylen, Amide wie z.B. N,N-Dimethylformamid oder N-Methylpyrrolidon oder aromatische Kohlenwasserstoffe wie z.B. Benzol, Toluol, Xylole, Mesitylen, Anisol, Phenetol oder Tetrahydronaphthalin. Weiterhin können auch gesättigte Kohlenwasserstoffe, wie Cyclohexan, n-Hexan oder n-Octan, Ester wie Methylacetat, Ethylacetat, Propylacetat oder Butylacetat oder Ether wie Diethylether, Methyl-tert-butylether, Tetrahydrofuran oder Dioxan verwendet werden. Bevorzugte Lösungsmittel für das erfindungsgemäße Verfahren sind Dichlormethan, Trichlormethan, Dichlorethylen oder Trichlorethylen, N,N-Dimethylformamid, N-Methylpyrrolidon, Benzol, Toluol, insbesondere Dichlormethan, Trichlormethan, Dichlorethylen oder Trichlorethylen.
Mischungen der genannten Lösungsmittel können ebenfalls Verwendung finden. Es ist ebenfalls möglich, die oben genannten Basen als Lösungsmittel zu verwenden.

Die Menge des Lösungsmittels ist nicht kritisch, im allgemeinen können 10 bis 10000 g Lösungsmittel je Mol des umzusetzenden Phenols der Formel II zugesetzt werden.

Generell sollten möglichst wasserfreie Lösungsmittel zum Einsatz kommen.
Nur bei Anwendung entsprechend größerer Mengen Perfluor-n-alkansulfonylfluorid, -chlorid oder -anhydrid und der jeweiligen Base kann in der Reaktionsmischung vorhandenes Wasser vernachlässigt werden.

Die Verbindungen der Formel l können z. B. nach folgendem Schema erhalten werden: 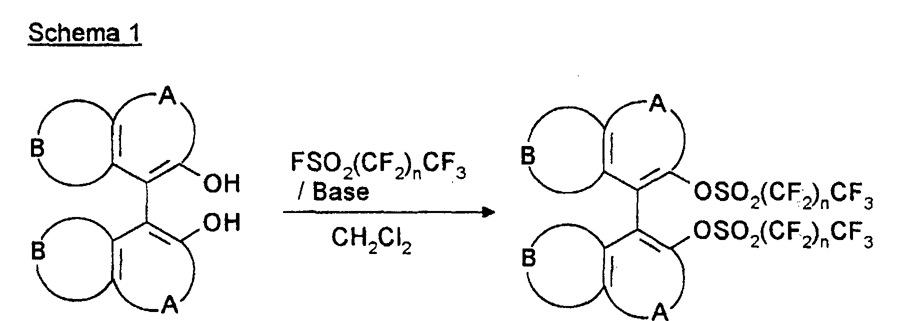 worin A, B und n die oben angegebene Bedeutung aufweisen.

Ein weiterer Gegenstand der Erfindung ist die Verwendung der Bis-(perfluor-n-alkansulfonate) der Formel l als Vorstufe für die Herstellung von chiralen Phosphan-Verbindungen der Formel III 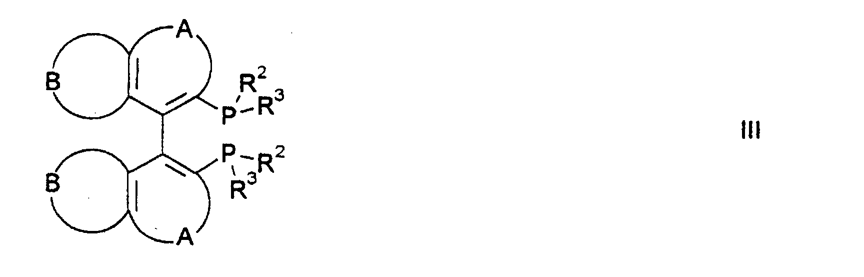 worin A und B die oben angegebene Bedeutung aufweisen
und
- R², R³: unabhängig voneinander Phenyl, 4-Methylphenyl, 3-Methylphenyl, 2-Methylphenyl, 3,5-Di methylphenyl, 3,5-Di-tert-butylphenyl, 4-Methoxyphenyl, 3-Methoxyphenyl, 2-Methoxyphenyl, 3,5-Dimethoxyphenyl, Cyclohexyl oder Cyclopentyl bedeutet,
sowie ein Verfahren zur Herstellung der Verbindungen der Formel III, dadurch gekennzeichnet, daß die Verbindungen der Formel l in Gegenwart eines Übergangsmetallkatalysators und einer Base entweder mit Phosphanen der Formel IV 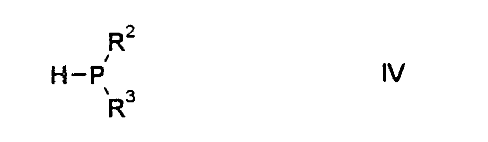 oder mit Zink und Phosphanen der Formel V 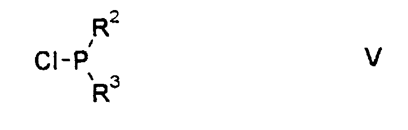 worin R² und R³ die oben angegebene Bedeutung aufweisen, umgesetzt werden.

Die Verbindungen der Formel III werden insbesondere als chirale Liganden für Übergangsmetallkatalysatoren eingesetzt, mit deren Hilfe es möglich ist, enantioselektive Reaktionen wie z.B. Hydrierungen oder C,C-Verknüpfungen durchzuführen.

Die bevorzugten Bedeutungen der Gruppen A und B, die für die Verbindungen der Formel l angegeben sind, gelten auch für die Verbindungen der Formel II und III.

Vorzugsweise bedeuten R² und R³ unabhängig voneinander Phenyl, 4-Methylphenyl, 3-Methylphenyl, 3,5-Dimethylphenyl, 3,5-Di-tert-butylphenyl oder Cyclohexyl. Insbesondere bevorzugt für R² und R³ ist die Bedeutung Phenyl. Verbindungen der Formel III, worin R² und R³ dieselbe Bedeutung annehmen, sind ganz besonders bevorzugt.

Besonders bevorzugte Verbindungen der Formel III, die unter Verwendung der Verbindungen der Formel I hergestellt werden können, sind die der Teilformeln lll1 - lll16: 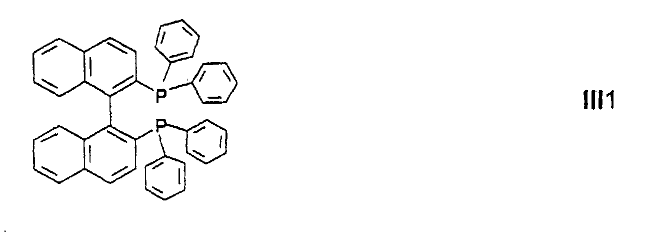 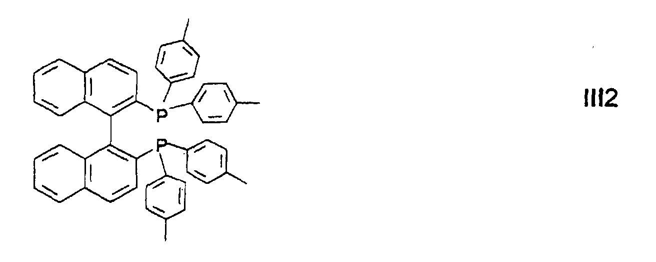 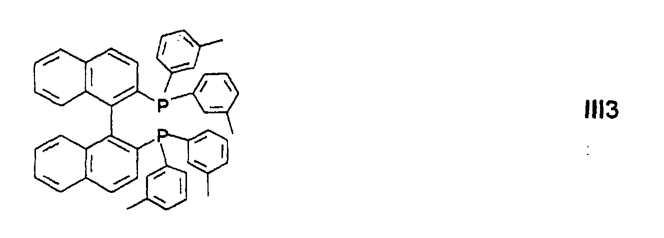 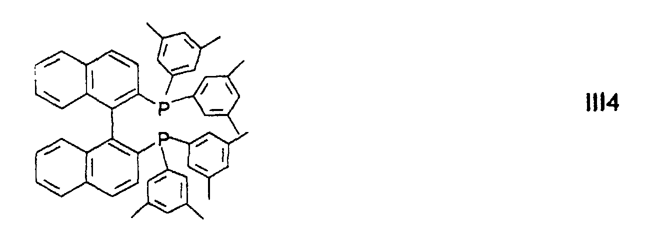 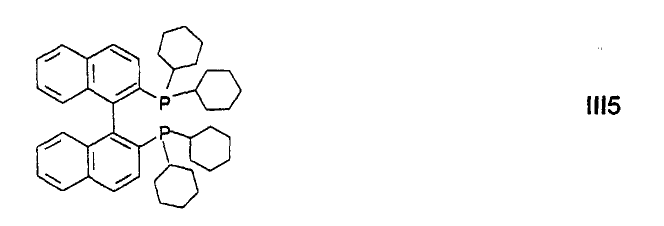 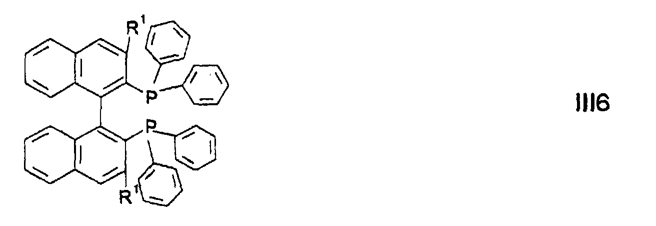 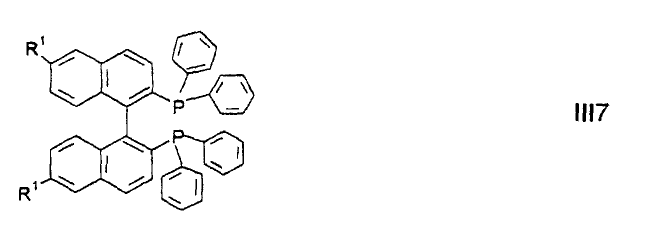 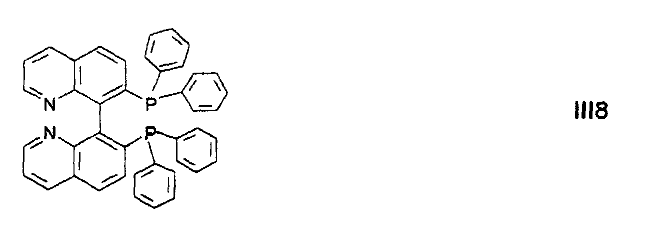 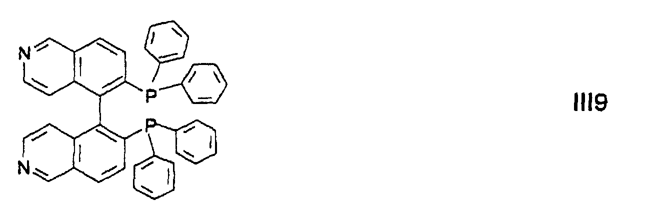 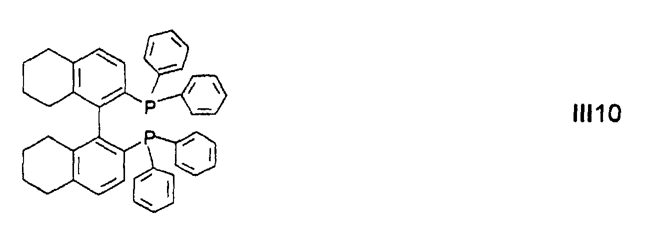 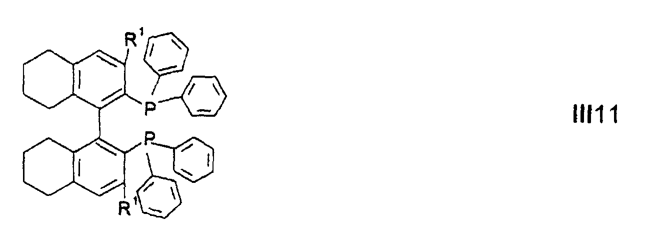 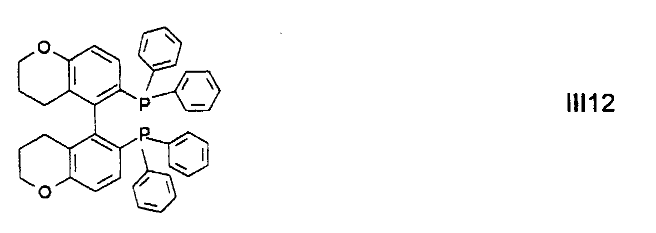 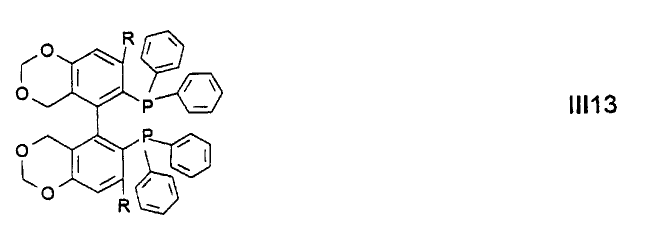 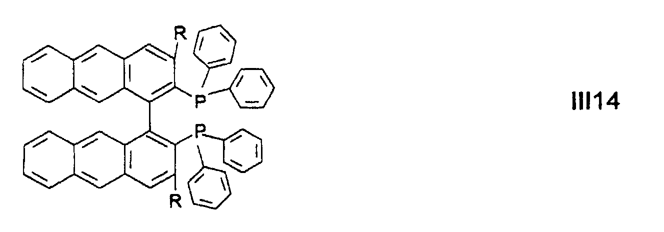 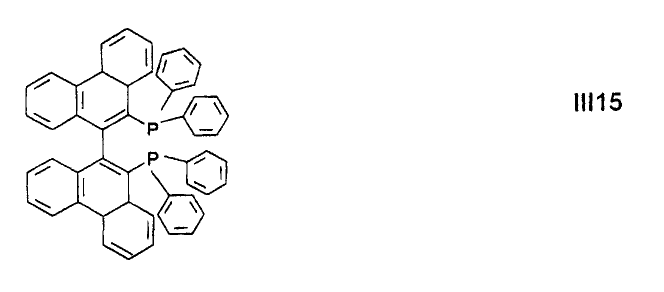 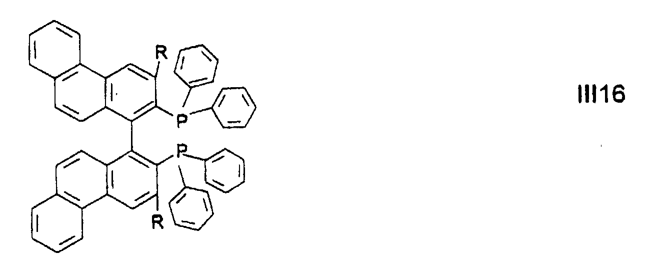 worin R und R¹ die oben angegebene Bedeutung annimmt.

Die Verbindungen der Formel III können in der Weise hergestellt werden, daß man die Perfluor-n-alkansulfonate der Formel l vorzugsweise in einem organischen Lösungsmittel bei Temperaturen von 20 °C bis 150°C, bevorzugt bei 30° - 120 °C, insbesondere bei 40 ° bis +100 °C bei erhöhtem oder verringertem Druck, vorzugsweise bei normalem Druck in Gegenwart einer Base und eines Übergangsmetallkatalysators mit einem Phosphan der Formel IV zur Reaktion bringt.

Die Verbindungen der F ormel III können auch in der Weise hergestellt werden, daß man die Perfluor-n-alkansulfonate der Formel l vorzugsweise ohne Lösungsmittel bei Temperaturen von 20 °C bis 150°C, bevorzugt bei 30° - 120 °C, insbesondere bei 40 ° bis +100 °C bei erhöhtem oder verringertem Druck, vorzugsweise bei normalem Druck in Gegenwart einer Base, Zink und eines Übergangsmetallkatalysators mit einem Phosphan der Formel V zur Reaktion bringt.

Zink wird bevorzugt in Form eines feinen Pulvers verwendet. Das molare Verhältnis des jeweiligen Perfluor-n-alkansulfonats der Formel l zu eingesetztem Zink beträgt für das erfindungsgemäße Verfahren zur Herstellung der Verbindungen der Formel III im allgemeinen 1 : 2 bis 1 : 40, bevorzugt 1 : 2 bis 1 : 20. Insbesondere ist ein Verhältnis von 1 : 2 bis 1 : 10 bevorzugt.

Das molare Verhältnis des jeweiligen Perfluor-n-alkansulfonats der Formel l zu den Phosphanen der Formel IV oder V beträgt für die erfindungsgemäßen Verfahren zur Herstellung der Verbindungen der Formel III im allgemeinen 1 : 2 bis 1 : 20, bevorzugt 1 : 2 bis 1 : 10. Insbesondere ist ein Verhältnis von 1 : 2 bis 1 : 5 bevorzugt.

Das molare Verhältnis des jeweiligen Perfluor-n-alkansulfonats der Formel l zur eingesetzten Base beträgt im allgemeinen 1 : 2 bis 1 : 20, bevorzugt 1 : 2 bis 1 : 15. Insbesondere ist ein Verhältnis von 1 : 2 bis 1 : 10 bevorzugt.

Das molare Verhältnis des jeweiligen Perfluor-n-alkansulfonats der Formel l zum eingesetzten Übergangsmetallkatalysator beträgt im allgemeinen 100 : 1 bis 2 : 1, bevorzugt 50 : 1 bis 5 : 1. Insbesondere ist ein Verhältnis von 20 : 1 bis 10 : 1 bevorzugt.

Als Übergangsmetallkatalysatoren für die Umsetzung der Verbindungen der Formel l zu den Phosphanen der Formel III kommen vorzugsweise Nickel- und Palladiumkatalysatoren wie z.B. Palladiumacetat, PdCl₂, PdCl₂-bis(triphenylphosphin) oder Pd-tetrakis(triphenylphosphin) zum Einsatz. Bevorzugt werden Nickelkatalysatoren verwendet. Besonders bevorzugt werden NiCl₂-bis(diphenyl)phosphinylmethan, -ethan, -propan oder -butan, NiBr₂, NiCl₂, NiCl₂-bis(diphenyl)phosphinylferrocen, NiCl₂-bis(triphenylphosphin), Ni-tetrakis(triphenylphosphin), Ni-tetrakis(triphenylphosphit) oder Ni-dicarbonylbis(triphenylphosphin) eingesetzt. Ganz besonders bevorzugt sind NiCl₂, NiCl₂-bis(diphenyl)phosphinylethan oder NiCl₂-bis(diphenyl)phosphinylpropan. Die Katalysatoren können auch in situ gebildet werden, derart, daß das Übergangsmetall oder Übergangsmetallsalz und der entsprechende Ligand getrennt der Reaktionsmischung zugesetzt werden. Es ist ebenfalls möglich, Mischungen der Übergangsmetallkatalysatoren der Reaktionsmischung zuzusetzen.

Geeignete Basen für die Umsetzung der Verbindungen der Formel l zu den Phosphanen der Formel III sind beispielsweise Alkali- und Erdalkalicarbonate wie z.B. Natrium-, Kalium-, Magnesium- oder Calciumcarbonat. Besonders geeignet sind Stickstoffheterocyclen, Amine oder Amidine. Vorzugsweise werden solche Stickstoffbasen eingesetzt, worin keine H-Atome direkt an ein N-Atom gebunden sind. Bevorzugte Stickstoffbasen sind Pyridine, Pyrimidine, Pyridazine, Trialkylamine Dialkylarylamine oder DABCO (Diazabicyclo[2.2.2]octan), wobei die Alkylreste in den Trialkylaminen und Dialkylarylaminen identisch oder unterschiedlich sein können. Insbesondere bevorzugt sind DABCO, Imidazol, Pyridin, p-Dimethylaminopyridin, m-Dimethylaminopyridin, o-Dimethylaminopyridin, Pyrimidin, Trimethylamin, Triethylamin, Tripropylamin, Triisopropylamin, Dimethylanilin, Diethylanilin. Ganz besonders bevorzugt sind DABCO, Pyridin, Imidazol, p-Dimethyl-aminopyridin, m-Dimethylaminopyridin, Trimethylamin, Triethylamin und Dimethylanilin. Es können auch Gemische der genannten Stickstoffbasen Verwendung finden.

Die Dauer der Reaktion beträgt im allgemeinen 1 Stunde bis 6 Tage.

Vorzugsweise werden polare Lösungsmittel für die Umsetzung der Verbindungen der Formel l zu den Phosphanen der Formel lll verwendet. Geeignete Lösungsmittel sind z.B. Amide wie z.B. N,N-Dimethylformamid oder N-Methylpyrrolidon, Sulfolan, Sulfoxide wie Dimethyl-, Diethyl- oder Dibutylsulfoxid, Nitrile wie Acetonitril oder Propionitril, Ester wie Methylacetat oder Ethylacetat oder Ether wie Tetrahydrofuran oder Dioxan oder Ketone wie z.B. Aceton. Bevorzugte Lösungsmittel für das erfindungsgemäße Verfahren sind N,N-Dimethylformamid, N-Methylpyrrolidon, Sulfolan, Dimethylsulfoxid oder Acetonitril.
Mischungen der genannten Lösungsmittel können ebenfalls Verwendung finden. Es ist ebenfalls möglich, die oben genannten Stickstoffbasen als Lösungsmittel zu verwenden.

Die Menge des Lösungsmittels ist nicht kritisch, im allgemeinen können 10 bis 10000 g Lösungsmittel je Mol des umzusetzenden Perfluor-n-alkansulfonats zugesetzt werden.
Die Verbindungen der Formel III können z. B. nach folgenden Schemata erhalten werden: 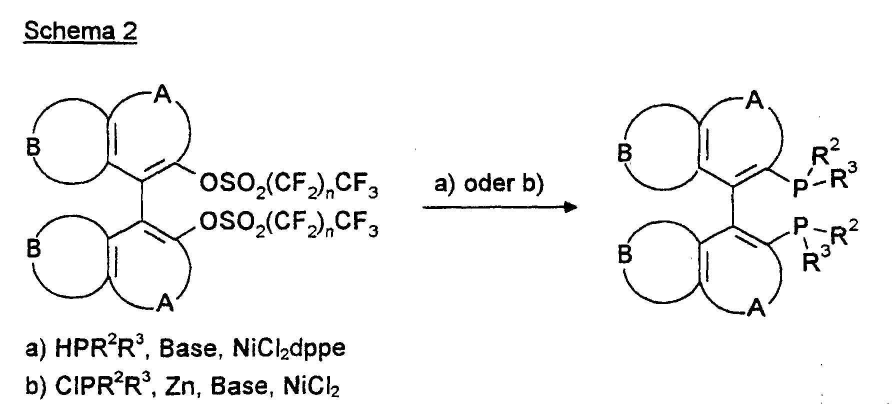 worin A, B, n, und R² und R³ die oben angegebene Bedeutung aufweisen.

Die Formeln l, II und III schließen sowohl die R- als auch die S-Enantiomeren der Verbindungen ein. Ebenso sind die Gemische der Enantiomeren von diesen Formeln umfaßt.

Die als Ausgangsstoffe benötigten Verbindungen der Formel II, IV und V sind entweder bekannt oder werden nach an sich bekannten Methoden hergestellt, wie sie in der Literatur beschrieben sind (z.B. in den Standardwerken wie Houben-Weyl, Methoden der Organischen Chemie, Georg-Thieme-Verlag, Stuttgart) und zwar unter Reaktionsbedingungen, die für die genannten Umsetzungen bekannt und geeignet sind. Dabei kann man auch von an sich bekannten, hier nicht näher erwähnten Varianten Gebrauch machen.

Auch ohne weitere Ausführungsformen wird davon ausgegangen, daß ein Fachmann die obige Beschreibung im weitesten Umfang nutzen kann. Die bevorzugten Ausführungsformen sind deswegen lediglich als beschreibende, keineswegs als in irgendeiner Weise limitierende Offenbarung aufzufassen.

Das folgende Beispiel soll die Erfindung erläutern, ohne sie zu begrenzen. Vor- und nachstehend bedeuten Prozentangaben Gewichtsprozent. Alle Temperaturen sind in Grad Celsius angegeben. Der Ausdruck "Raumtemperatur" bezieht sich auf 20 °C.

### Beispiel 1

### Racemisches Binaphtholdinonaflat (Binaphtholbis(nonafluor-nbutansulfonat))

Zu einer Lösung von 45,6 g racemischem Binaphthol (160 mmol) und 47,6 g Triethylamin (470 mmol; 65,5 ml) in 350 ml Dichlormethan gab man bei Raumtemperatur 111,6 g Nonafluor-n-butansulfonylfluorid (370 mmol). Nach wenigen Minuten war die dichtere Sulfonylfluorid-Phase verschwunden, ohne daß sich die Mischung nennenswert erwärmte. Nach vierstündigem Rühren wurde einmal mit 80 ml Natronlauge (5 Gew.-%) und einmal mit 80 ml Wasser ausgeschüttelt, die organische Phase über Natriumsulfat getrocknet und einrotiert. Das Produkt kristallisierte beim Abkühlen aus und wurde einer weiteren Reinigung durch Waschen mit einer kleinen Menge kaltem Methanol und nachfolgendes Absaugen unterzogen. Erhalten wurden 123 g Binaphtholdinonaflat (90,0 %) als farblose Kristalle vom Schmelzpunkt 104,1 °C; HPLC-Reinheit > 99 % (RP-18; Methanol/Wasser 80:20; 254 nm).

### Beispiel 2

### (R)- bzw. (S)-Binaphtholdinonaflat

Die Präparation von (R)- bzw. (S)-Binaphtholdinonaflat erfolgte nach der für das entsprechende Racemat angegebenen Vorschrift unter Verwendung von (R)- bzw. (S)-Binaphthol, das z. B. nach USP 5399771 erhältlich ist. Einziger Unterschied ist die honigartige Konsistenz der reinen Enantiomeren von Binaphtholdinonaflat, die in Kombination mit einer viel höheren Löslichkeit in Methanol den Aufreinigungsschritt durch Waschen mit diesem Lösungsmittel verunmöglicht. Erhalten wurden 95 % d. Th. einer hochviskosen Flüssigkeit, die nach mehrwöchigem Stehen bei Raumtemperatur allmählich kristallisierte (HPLC-Reinheit > 98 %; RP-18; Methanol/Wasser 80:20). Die Enantiomerenreinheit wurde mittels HPLC bestimmt und entsprach exakt der des eingesetzten Binaphthols (Chiradex 10 µm; Lösung in Methanol; c = 1 mg/ml; CH₃OH/H₂O 70:30; 254 nm). [α]^{D} = -87,4° ((R)-Binaphtholdinonaflat; c = 1 THF; 20 °C).

Analog wurden aus den entsprechenden Vorstufen die folgenden erfindungsgemäßen Verbindungen erhalten:

### Beispiele 3 - 15

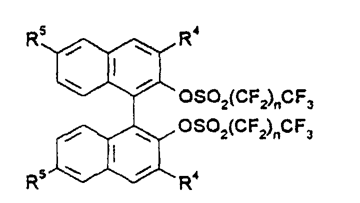

| | n | R⁴ | R⁵ |
|---|---|---|---|
| (3) | 3 | Methyl | H |
| (4) | 3 | Ethyl | H |
| (5) | 3 | Methoxy | H |
| (6) | 3 | CF₃ | H |
| (7) | 7 | CN | H |
| (8) | 7 | Phenyl | H |
| (9) | 3 | F | Methyl |
| (10) | 3 | CN | Methoxy |
| (11) | 4 | H | Methyl |
| (12) | 5 | H | Pentyl |
| (13) | 7 | H | Methoxy |
| (14) | 3 | H | CN |
| (15) | 3 | H | Phenyl |

### Beispiele 16 - 23

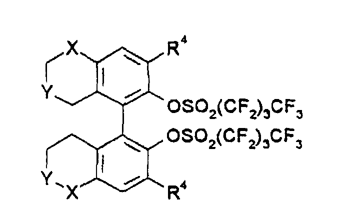

| | R⁴ | X | Y |
|---|---|---|---|
| (16) | H | CH₂ | CH₂ |
| (17) | CN | CH₂ | CH₂ |
| (18) | CF₃ | CH₂ | CH₂ |
| (19) | Methoxy | CH₂ | CH₂ |
| (20) | H | O | CH₂ |
| (21) | CN | O | CH₂ |
| (22) | Methyl | O | O |
| (23) | CF₃ | O | O |

### Beispiele 24 - 26

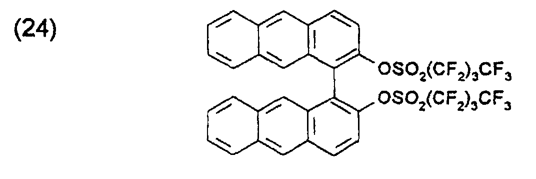

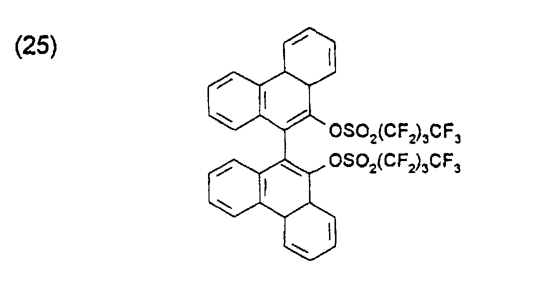

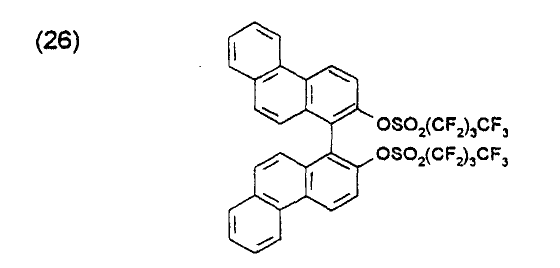

### Beispiel 27

Zu einer Suspension von 663 mg NiCl₂(dppe) in 25 ml DMF gab man bei Raumtemperatur und unter Argon 1,25 ml Diphenylphosphin. Nach halbstündigem Rühren bei 100 °C fügte man im Argon-Gegenstrom mittels einer Spritze mit aufgesetzter Injektionskanüle eine Lösung von 5,63 g DABCO und 10,6 g enantiomerenreinem (R)- bzw. (S)-Binaphtholdinonaflat oder racemischen Binaphtholdinonaflat (125 mmol) in 37,5 ml DMF hinzu. Die Reaktionsmischung wurde weiter bei 100 °C belassen; nach einer, drei und sieben Stunden fügte man jeweils 1,25 ml Diphenylphosphin hinzu. Das Fortschreiten der Reaktion wurde per HPLC verfolgt (Purospher RP-18; Acetonitril/Wasser 90:10). Nach dem vollständigen Verbrauch des Eduktes (etwa zwei Tage; die Reaktion läßt sich durch gelegentliche Zugabe von jeweils 0,15 g DABCO und 50 mg NiCl₂(dppe) beschleunigen) ließ man die Reaktionsmischung erkalten. Nach Absaugen und Waschen mit wenig kaltem Methanol erhielt man (R)-, (S)- oder racemisches 2,2'-Bis(diphenylphosphin)-1,1'-binaphthyl (BINAP) als schneeweißes, feinkristallines Pulver in einer Ausbeute von 77 % (8,1 g; 125 mmol).

### Beispiel 28

Eine Reaktionsmischung aus 400 mg Zinkstaub, 300 mg DABCO, 25 mg Nickel(ll)chlorid, 1,0 g racemischem oder enantiomerenreinem Binaphtholdinonaflat und 0,30 ml Chlordiphenylphosphin wurde unter Schutzgas auf 100 °C erwärmt. Das Fortschreiten der Reaktion wurde durch HPLC-Kontrolle verfolgt; alle drei Stunden gab man jeweils weitere 25 mg NiCl₂ und erforderlichenfalls auch Chlordiphenylphosphin hinzu.

Nach dem vollständigen Verbrauch des Eduktes ließ man die Reaktionsmischung erkalten. Der Niederschlag wurde abgesaugt und mit siedendem Toluol ausgekocht. Nach Schutzgasfiltration und Abkühlen auf 5 °C wurde (R)-, (S)- oder racemisches BINAP als schneeweißes, feinkristallines Pulver erhalten.

Analog wurden aus den entsprechenden Vorstufen die folgenden Verbindungen erhalten:

### Beispiele 29 - 41

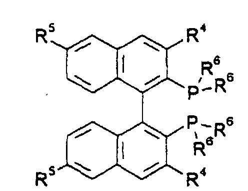

| | R⁶ | R⁴ | R⁶ |
|---|---|---|---|
| (29) | Phenyl | Methyl | H |
| (30) | 4-Methylphenyl | Ethyl | H |
| (31) | Phenyl | Methoxy | H |
| (32) | Phenyl | CF₃ | H |
| (33) | Phenyl | CN | H |
| (34) | Phenyl | Phenyl | H |
| (35) | Cyclohexyl | F | Methyl |
| (36) | 3-Methylphenyl | CN | Methoxy |
| (37) | Phenyl | H | Methyl |
| (38) | 4-Methylphenyl | H | Pentyl |
| (39) | Cyclohexyl | H | Methoxy |
| (40) | Phenyl | H | CN |
| (41) | Phenyl | H | Phenyl |

### Beispiele 42 - 49

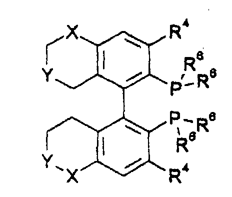

| | R⁵ | R⁴ | X | Y |
|---|---|---|---|---|
| (42) | Phenyl | H | CH₂ | CH₂ |
| (43) | Phenyl | CN | CH₂ | CH₂ |
| (44) | Phenyl | CF₃ | CH₂ | CH₂ |
| (45) | 4-Methylphenyl | Methoxy | CH₂ | CH₂ |
| (46) | 3-Methylphenyl | H | O | CH₂ |
| (47) | Cyclohexyl | CN | O | CH₂ |
| (48) | Cyclopentyl | Methyl | O | O |
| (49) | Phenyl | CF₃ | O | O |

### Beispiele 50- 52

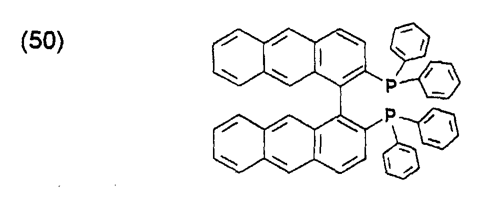

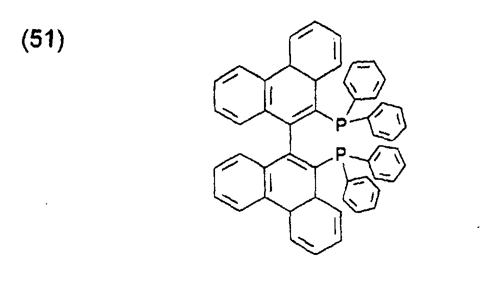

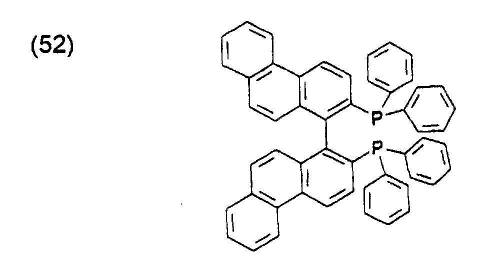

## Patentansprüche

1. Bis(perfluor-n-alkansulfonate) der Formel l: 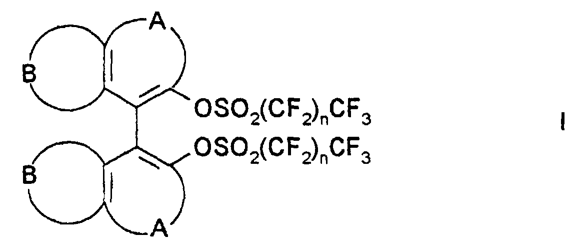 worin
n 3, 4, 5, 6, 7, 8 oder 9,
A 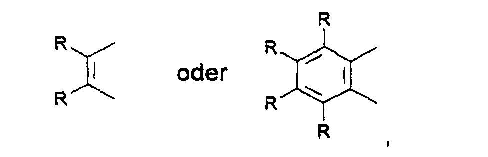
B -(CHR)₄-, 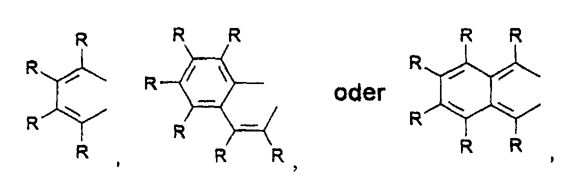 wobei nicht benachbarte Gruppen =CR- durch =N- und ferner -CHRdurch -NR-, -O- oder -S- ersetzt sein können
und
R Alkyl oder Alkoxy mit 1 bis 12 C-Atomen, Halogen, -CN, -CF₃, -OCF₃ oder unsubstituiertes oder einfach oder mehrfach durch Alkyl oder Alkoxy mit 1 bis 12 C-Atomen, Halogen oder -CN substituiertes Phenyl, wobei R bei mehrfachem Auftreten dieselbe oder verschiedene Bedeutungen aufweisen kann,
bedeuten.

2. Verbindungen der Formel l nach Anspruch 1, **dadurch gekennzeichnet, daß** A 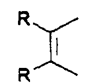 bedeutet und B die Bedeutung -(CHR₂)₄- oder 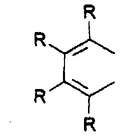 aufweist.

3. Verbindungen der Formel I nach Anspruch 1 oder 2, **dadurch gekennzeichnet, daß** R Alkyl oder Alkoxy mit 1 bis 7 C-Atomen, F, Br, CN, -CF₃, -OCF₃ bedeutet.

4. Verbindungen der Formeln l1, l2, l3, l7, und l8: 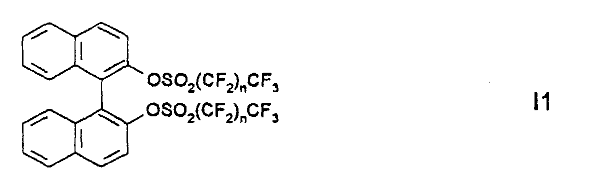 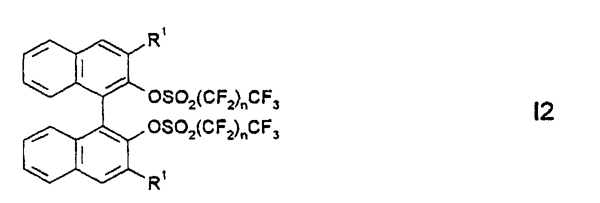 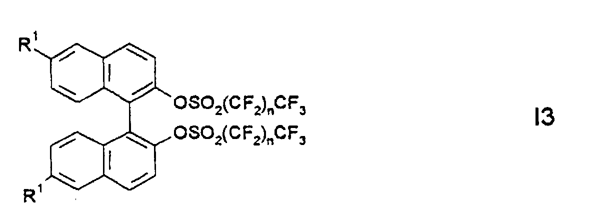 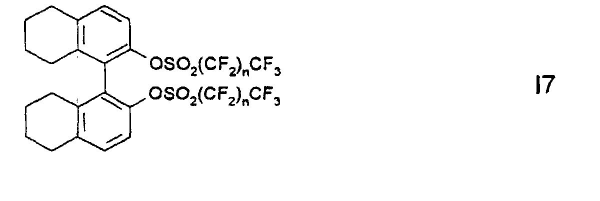 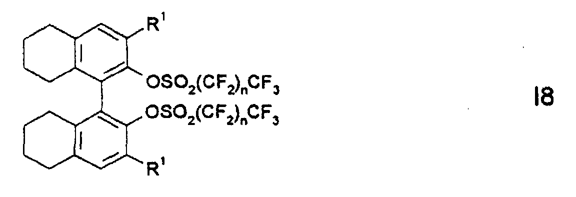 worin n die oben angegebene Bedeutung aufweist und R¹ Alkyl oder Alkoxy mit 1 bis 3 C-Atomen, F, Br, CF₃ oder CN bedeutet.

5. Verfahren zur Herstellung der Bis(perfluor-n-alkansulfonate) der Formel l, **dadurch gekennzeichnet, daß** man die Verbindungen der Formel II: 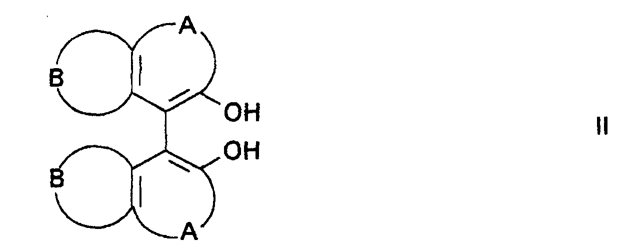 worin A und B die in Anspruch 1 angegebene Bedeutung aufweisen, mit Perfluor-n-alkansulfonylfluorid, -chlorid oder -anhydrid in Gegenwart einer Base umsetzt.

6. Verfahren zur Herstellung der Verbindungen der Formel l nach Anspruch 5, **dadurch gekennzeichnet, daß** man die Verbindungen der Formel ll mit Nonafluor-n-butansulfonylfluorid oder Perfluorn-oktansulfonylfluorid in Gegenwart einer Base umsetzt.

7. Verfahren zur Herstellung der Verbindungen der Formel l nach Anspruch 5 oder 6, **dadurch gekennzeichnet, daß** man als Base Pyridine, Pyrimidine, Pyridazine, Trialkylamine oder Dialkylarylamine verwendet.

8. Verwendung der Bis(perfluor-n-alkansulfonate) der Formel l zur Herstellung von Diphosphanen der Formel III: 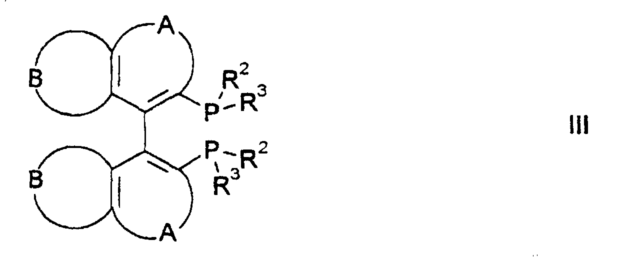 worin A und B die oben angegebene Bedeutung aufweisen
und
R², R³ Phenyl, 4-Methylphenyl, 3-Methylphenyl, 2-Methylphenyl, 3,5-Dimethylphenyl, 3,5-Di-tert-butylphenyl, 4-Methoxyphenyl, 3-Methoxyphenyl, 2-Methoxyphenyl, 3,5-Dimethoxyphenyl, Cyclohexyl oder oder Cyclopentyl bedeuten.

9. Verfahren zur Herstellung der Verbindungen der Formel III, **dadurch gekennzeichnet, daß** die Verbindungen der Formel l in Gegenwart eines Übergangsmetallkatalysators und einer Base entweder mit Phosphanen der Formel IV 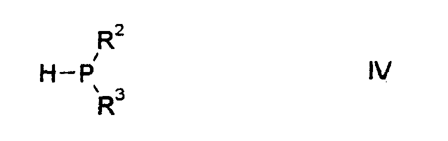 oder mit Zink und Phosphanen der Formel V 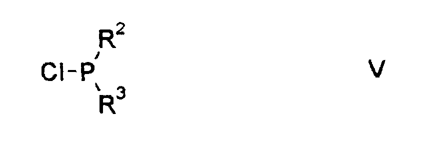 worin R² und R³ die oben angegebene Bedeutung aufweisen, umgesetzt werden.

10. Verfahren zur Herstellung der Verbindungen der Formel III nach Anspruch 9, **dadurch gekennzeichnet, daß** als Übergangsmetallkatalysator ein Nickel-Katalysator eingesetzt wird.

## Claims

1. Bis(perfluoro-n-alkanesulfonates) of the formula I: 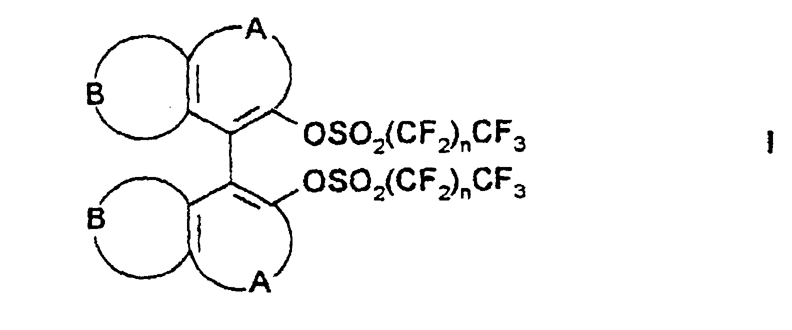 where
n is 3, 4, 5, 6, 7, 8 or 9,
A is 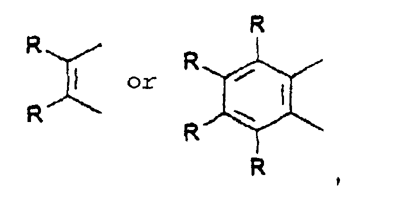
B is -(CHR)₄-, 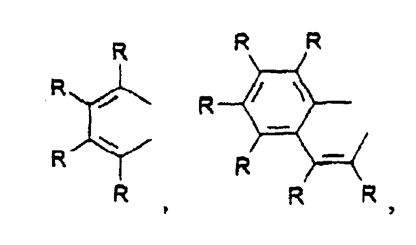 or 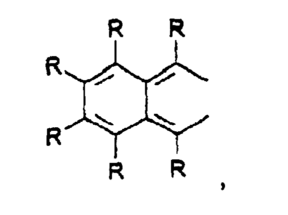 where nonadjacent groups =CR- may be replaced by =N-, and -CHR- may be replaced by -NR-, -O- or -S-
and
R is alkyl or alkoxy having from 1 to 12 carbon atoms, halogen, -CN, -CF₃, -OCF₃ or unsubstituted phenyl or phenyl which is monosubstituted or polysubstituted by alkyl or alkoxy having from 1 to 12 carbon atoms, halogen or -CN, where if more than one R is present the substituents R may be identical or different.

2. Compounds of the formula I according to Claim 1, **characterized in that** A is 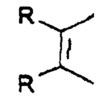 and B is -(CHR₂)₄- or

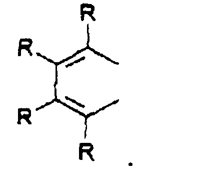

3. Compounds of the formula I according to Claim 1 or 2, **characterized in that** R is alkyl or alkoxy having from 1 to 7 carbon atoms, F, Br, CN, -CF₃, -OCF₃.

4. Compounds of the formulae I1, I2, I3, I7 and I8: 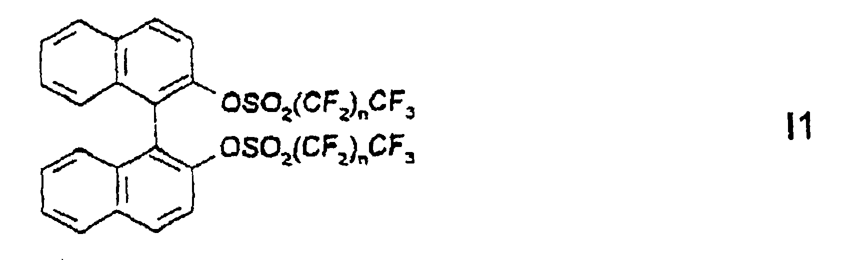 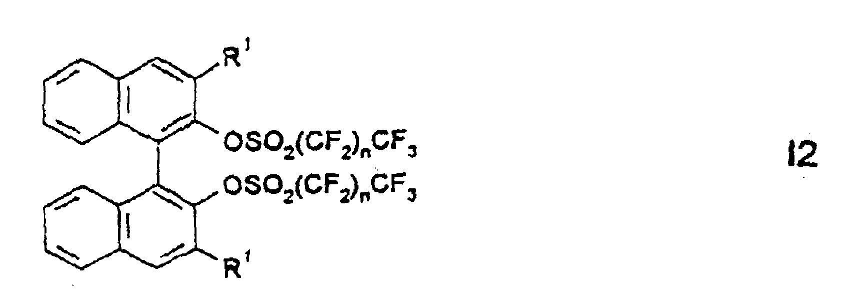 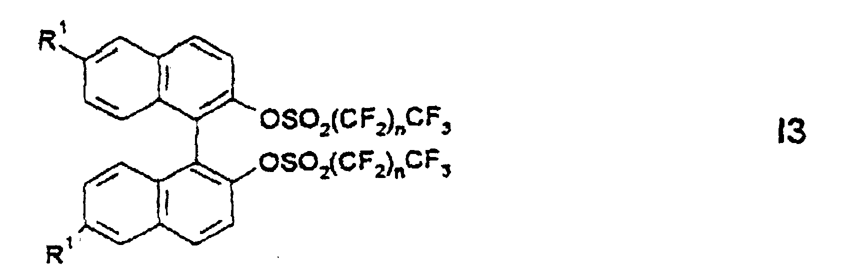 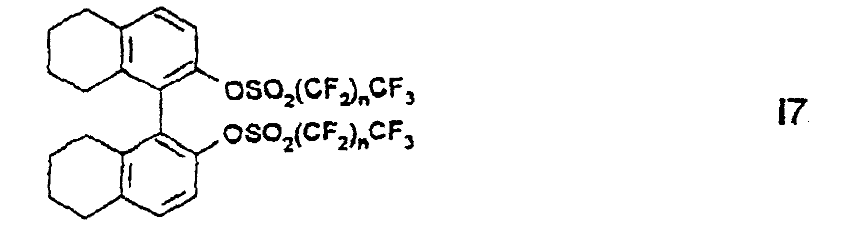 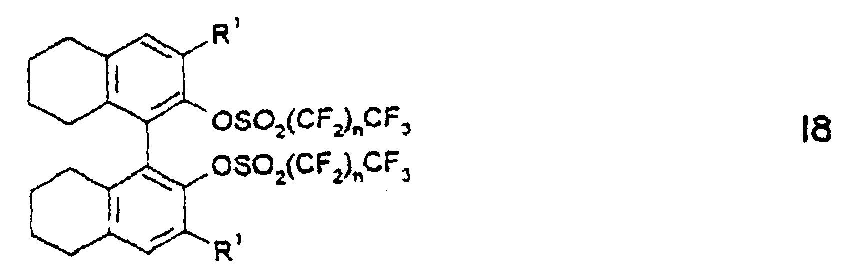 where n is as defined above and R¹ is alkyl or alkoxy having from 1 to 3 carbon atoms, F, Br, CF₃ or CN.

5. Process for preparing the bis(perfluoro-n-alkanesulfonates) of the formula I, **characterized in that** the compounds of the formula II: 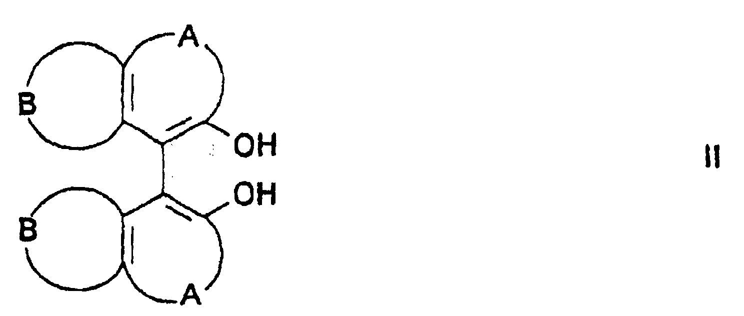 where A and B are as defined in Claim 1 are reacted with perfluoro-n-alkanesulfonyl fluoride, chloride or anhydride in the presence of a base.

6. Process for preparing the compounds of the formula I according to Claim 5, **characterized in that** the compounds of the formula II are reacted with nonafluoro-n-butanesulfonyl fluoride or perfluoro-n-octanesulfonyl fluoride in the presence of a base.

7. Process for preparing the compounds of the formula I according to Claim 5 or 6, **characterized in that** the base used is a pyridine, a pyrimidine, a pyridazine, a trialkylamine or a dialkylarylamine.

8. Use of the bis(perfluoro-n-alkanesulfonates) of the formula I for preparing diphosphines of the formula III: 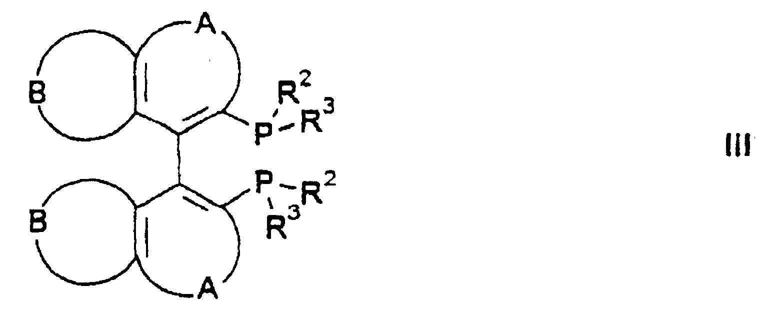 where A and B are as defined above
and
R²,R³ are phenyl, 4-methylphenyl, 3-methylphenyl, 2-methylphenyl, 3,5-dimethylphenyl, 3,5-di-tert-butylphenyl, 4-methoxyphenyl, 3-methoxyphenyl, 2-methoxyphenyl, 3,5-dimethoxyphenyl, cyclohexyl or cyclopentyl.

9. Process for preparing the compounds of the formula III, **characterized in that** the compounds of the formula I are reacted in the presence of a transition metal catalyst and a base either with phosphines of the formula IV 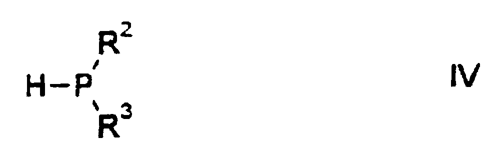 or with zinc and phosphines of the formula V 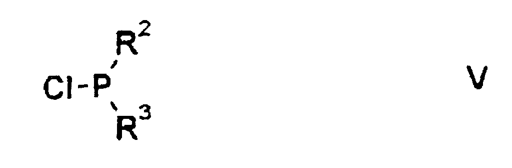 where R² and R³ are as defined above.

10. Process for preparing the compounds of the formula III according to Claim 9, **characterized in that** the transition metal catalyst used is a nickel catalyst.

## Revendications

1. Bis(perfluoro-n-alcanesulfonate)s de formule I 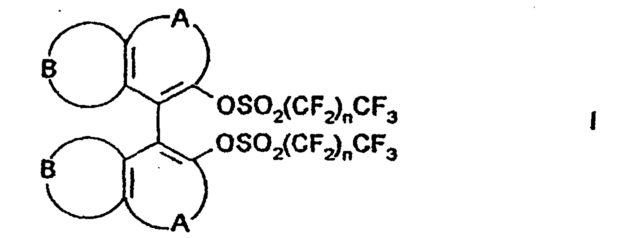 dans laquelle
n représente 3, 4, 5, 6, 7, 8 ou 9,
A représente 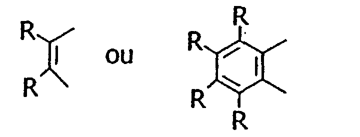
B représente -(CHR)₄-, 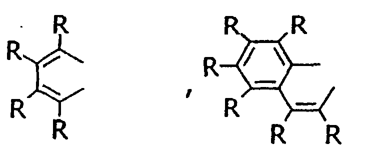 ou 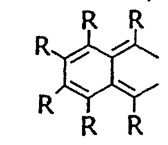 des groupes =CR- non contigus pouvant être remplacés par =N- et en outre -CHR- pouvant être remplacé par -NR-, -O- ou -S-
et
R représente un groupe alkyle ou alcoxy ayant de 1 à 12 atomes de carbone, un atome d'halogène ou un groupe -CN, -CF₃, -OCF₃ ou un groupe phényle non substitué ou une ou plusieurs fois substitué par un ou des groupes alkyle ou alcoxy ayant de 1 à 12 atomes de carbone, halogène ou -CN, R pouvant dans ses multiples occurrences avoir la même signification ou des significations différentes.

2. Composés de formule I selon la revendication 1, caractërisés en ce que A représente 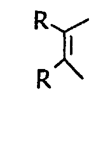 et B a la signification de -(CH₂)₄ ou

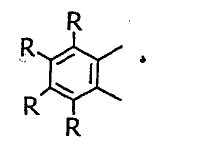

3. Composés de formule I selon la revendication 1 ou 2, **caractérisés en ce que** R représente un groupe alkyle ou alcoxy ayant de 1 à 7 atomes de carbone, F, Br, CN, -CF₃, -OCF₃.

4. Composés de formules 11, 12, 13, 17 et 18: 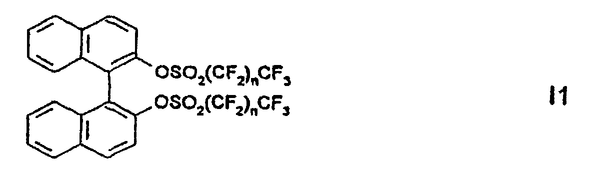 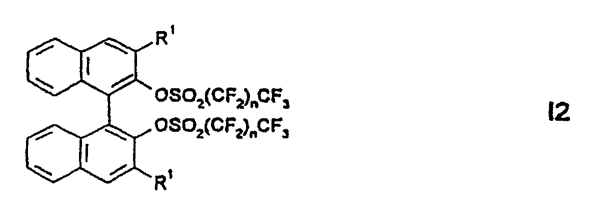 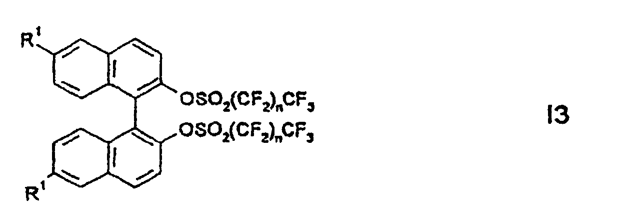 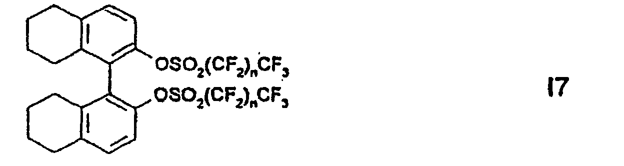 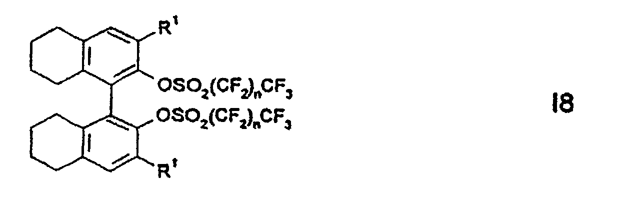 dans lesquelles n a la signification donnée plus haut et R¹ représente un groupe alkyle ou alcoxy ayant de 1 à 3 atomes de carbone, F, Br, CF₃ ou CN.

5. Procédé pour la préparation des bis(perfluoro-n-alcanesulfonate)s de formule I, **caractérisé en ce qu'**on fait réagir les composés de formule Il : 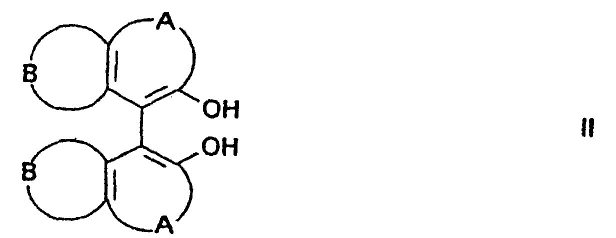 dans laquelle A et B ont les significations données dans la revendication 1, avec un fluorure, chlorure de perfluoro-n-alcanesulfonyle ou un anhydride perfluoro-n-alcanesulfonique, en présence d'une base.

6. Procédé pour la préparation des composés de formule I selon la revendication 5, **caractérisé en ce qu'**on fait réagir les composés de formule Il avec du fluorure de nonafluoro-n-butanesulfonyle ou du fluorure de perfluoro-n-octanesulfonyle en présence d'une base.

7. Procédé pour la préparation des composés de formule I selon la revendication 5 ou 6, **caractérisé en ce qu'**on utilise comme base des pyridines, pyrimidines, pyridazines, trialkylamines ou dialkylarylamines.

8. Utilisation des bis(perfluoro-n-alcanesulfonate)s de formule I pour la préparation de diphosphanes de formule III : 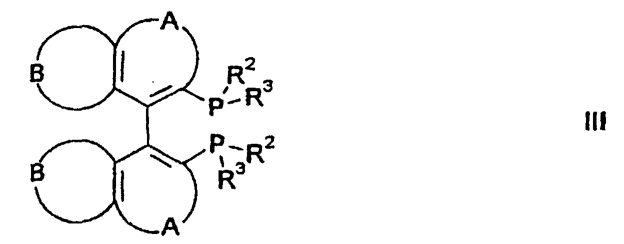 dans laquelle A et B ont les significations précédentes
et
R², R³ représentent le groupe phényle, 4-méthylphényle, 3-méthylphényle, 2-méthylphényle, 3,5-diméthylphényle, 3,5-di-tert-butylphényle, 4-méthoxyphényle, 3-méthoxyphényle, 2-méthoxyphényle, 3,5-diméthoxyphényle, cyclohexyle ou cyclopentyle.

9. Procédé pour la préparation des composés de formule III, **caractérisé en ce qu'**on fait réagir les composés de formule I en présence d'une base et d'un catalyseur à base d'un métal de transition, soit avec des phosphanes de formule IV 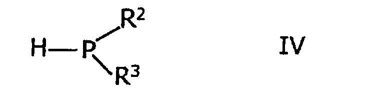 soit avec du zinc et des phosphanes de formule V 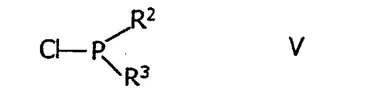 formules dans lesquelles R² et R³ ont les significations données plus haut.

10. Procédé pour la préparation des composés de formule III selon la revendication 9, **caractérisé en ce qu'**on utilise comme catalyseur à base d'un métal de transition un catalyseur à base de nickel.
